# EUROPEAN PATENT APPLICATION

(11) **EP 2 613 176 A2**
(43) Date of publication of application: **10.07.2013**
(21) Application number: 13150660.2
(22) Date of filing: 09.01.2013
(51) Int. Cl.: G01S 15/89

(54) **Ultrasonic device and method of generating ultrasonic image using vector doppler**

(30) Priority: 09.01.2012 US 201261584374 P; 09.07.2012 KR 20120074690
(71) Applicant: Samsung Electronics Co., Ltd., Gyeonggi-do 443-742 (KR); Industry-University Cooperation Foundation Sogang University, Seoul (KR)
(72) Inventor: Shim, Hwan, Gyeonggi-do (KR); Kim Gi-duck, Incheon (KR); Jeon, Kang-won, Gyeonggi-do (KR); Song, Tai-kyong, Seoul (KR); Yoon, Sung-soo, Seoul (KR); Yoo, Yang-mo, Gyeonggi-do (KR); Chang, Jin-ho, Seoul (KR); Kim, Young-tae, Gyeonggi-do (KR); Lim, Hyung-joon, Seoul (KR); Cheon, Byeong-geun, Gyeonggi-do (KR)
(74) Representative: Bray, Richard Anthony

(57) **Abstract**

Disclosed is a method for generating an ultrasonic image, the method including: transmitting an ultrasonic signal to a predetermined portion of an object and receiving at least three response signals which are reflected from the predetermined portion; selecting at least two response signals from among the at least three received response signals; and acquiring vector information which indicates a speed and a movement direction of the predetermined portion based on a receiving angle and a Doppler frequency of each of the selected at least two response signals.

## Description

### CROSS-REFERENCE TO RELATED PATENT APPLICATIONS

This application claims the benefit of U.S. Provisional Patent Application No. 61/584,374, filed on January 9, 2012, in the United States Patent and Trademark Office, and priority from Korean Patent Application No. 10-2012-0074690, filed on July 9, 2012, in the Korean Intellectual Property Office, the disclosures of which are incorporated herein by reference in their entireties.

### BACKGROUND

### 1. Field

Exemplary embodiments relate to a device and a method for generating an ultrasonic image by using vector Doppler.

### 2. Description of the Related Art

An ultrasonic device may be used to observe an internal structure of a body of an organism. The ultrasonic device, which is a noninvasive device, shows structural details, internal tissues, and fluid flow in the body.

The ultrasonic device may show blood flow or tissue movement on a color Doppler image or a Doppler spectrum image. From the color Doppler image or the Doppler spectrum image, an examiner may identify heart valve motion and blood flow velocity or a blood flow rate of an examinee.

The color Doppler image or Doppler spectrum image typically includes information relating to a speed and a movement direction of blood or tissue. A method for accurately measuring this information is required.

### SUMMARY OF THE INVENTION

Exemplary embodiments provide a device and a method for generating an ultrasonic image by using vector Doppler to accurately measure vector information relating to an object.

Exemplary embodiments also provide a device and a method for generating an ultrasonic image by using vector Doppler for providing a reliable color Doppler image or a Doppler spectrum image to an examiner.

According to the present invention there is provided an apparatus and method as set forth in the appended claims. Other features of the invention will be apparent from the dependent claims, and the description which follows.

According to an aspect of one or more exemplary embodiments, there is provided a method for generating an ultrasonic image, including: transmitting an ultrasonic signal to a predetermined portion of an object and receiving at least three response signals which are respectively reflected from the predetermined portion; selecting at least two response signals from among the at least three received response signals; and acquiring vector information which indicates a speed and a movement direction of the predetermined portion based on a respective receiving angle and a respective Doppler frequency of each of the selected at least two response signals.

The selecting the at least two response signals may include selecting the at least two response signals based on at least one of a respective receiving angle and a respective power of each of the at least three received response signals.

The selecting the at least two response signals may include selecting the at least two response signals from among the at least three response signals in a descending order of the powers of the at least three received response signals.

The acquiring the vector information may include acquiring the respective Doppler frequency of each of the selected at least two response signals by using a respective autocorrelation of ensemble signals for each of the selected at least two response signals.

According to another aspect of one or more exemplary embodiments, there is provided a method for generating an ultrasonic image, including: transmitting an ultrasonic signal to a predetermined portion of an object and receiving a first response signal which is reflected from the predetermined portion; acquiring vector information which indicates a speed and a movement direction of the predetermined portion based on a receiving angle and a Doppler frequency of the received first response signal; and adjusting, based on the acquired vector information, a steering angle of the ultrasonic signal.

The method may further include transmitting the ultrasonic signal to the object at the adjusted steering angle and receiving a second response signal which is reflected from the object, and generating at least one of a color Doppler image and a Doppler spectrum image of the object based on the received second response signal.

The method may further include displaying the generated at least one of the color Doppler image and the Doppler spectrum image.

According to another aspect of one or more exemplary embodiments, there is provided a method for generating an ultrasonic image, including: transmitting an ultrasonic signal to each of a plurality of predetermined portions which are included in a first region of interest of an object and receiving a respective plurality of response signals which are respectively reflected from each of the plurality of predetermined portions; acquiring a plurality of pieces of vector information which respectively indicate respective speeds and respective movement directions of each of the plurality of predetermined portions based on respective receiving angles and respective Doppler frequencies of each of the plurality of received response signals; determining a vector sum of the acquired plurality of pieces of vector information; and adjusting a steering angle of the ultrasonic signal based on the determined vector sum.

The method may further include determining a second region of interest of the object based on the adjusted steering angle.

According to another aspect of one or more exemplary embodiments, there is provided an ultrasonic device including: a probe which transmits an ultrasonic signal to a predetermined portion of an object and which receives at least three response signals which are respectively reflected from the predetermined portion; a controller which selects at least two response signals from among the at least three received response signals; and a vector information acquisition device which acquires vector information which indicates a speed and a movement direction of the predetermined portion based on a respective receiving angle and a respective Doppler frequency of each of the selected at least two response signals.

The controller may select the at least two response signals based on at least one of a respective receiving angle and a respective power of each of the at least three received response signals.

The controller may select the at least two response signals from among the at least three response signals in a descending order of the powers of the at least three received response signals.

The vector information acquisition device may acquire the respective Doppler frequency of each of the selected at least two response signals by using a respective autocorrelation of ensemble signals for each of the selected at least two response signals.

According to another aspect of one or more exemplary embodiments, there is provided an ultrasonic device including: a probe which transmits an ultrasonic signal to a predetermined portion of an object and receives a first response signal which is reflected from the predetermined portion; a vector information acquisition device which acquires vector information which indicates a speed and a movement direction of the predetermined portion based on a receiving angle and a Doppler frequency of the received first response signal; and a controller which adjusts, based on the acquired vector information, a steering angle of the ultrasonic signal.

The probe may transmit the ultrasonic signal to the object at the adjusted steering angle and receive a second response signal which is reflected from the object, and the ultrasonic device may further include an image generator which generates at least one of a color Doppler image and a Doppler spectrum image of the object by using the received second response signal.

The ultrasonic device may further include a display which displays the generated at least one of the color Doppler image and the Doppler spectrum image.

According to another aspect of one or more exemplary embodiments, there is provided an ultrasonic device including: a probe which transmits an ultrasonic signal to each of a plurality of predetermined portions which are included in a first region of interest of an object and which receives a respective plurality of response signals which are respectively reflected from each of the plurality of predetermined portions; a vector information acquisition device which acquires a plurality of pieces of vector information which respectively indicate respective speeds and respective movement directions of the plurality of predetermined portions based on respective receiving angles and respective Doppler frequencies of each of the plurality of received response signals, and which determines a vector sum of the plurality of pieces of vector information; and a controller which adjusts a steering angle of the ultrasonic signal based on the determined vector sum.

The controller may determine a second region of interest of the object based on the adjusted steering angle.

A program which includes instructions for causing a computer to execute at least one of the ultrasonic image generating methods described above may be recorded in a non-transitory computer-readable recording medium.

### BRIEF DESCRIPTION OF THE DRAWINGS

The above and other features and advantages will become more apparent by describing in detail exemplary embodiments with reference to the attached drawings in which:
FIG. 1 is a diagram which illustrates a conventional method for obtaining vector information relating to an object;
FIG. 2 is a diagram which illustrates a configuration of an ultrasonic device according to an exemplary embodiment;
FIG. 3 is a diagram which illustrates a configuration of an ultrasonic device according to another exemplary embodiment;
FIG. 4 is a diagram which illustrates a method for obtaining vector information relating to a predetermined portion of an object by using an ultrasonic device according to an exemplary embodiment of the present invention;
In FIG. 5, drawings (a) and (b) are diagrams which illustrate a method for adjusting a steering angle of an ultrasonic device based on vector information relating to a predetermined portion of an object;
In FIG. 6, drawings (a) and (b) are diagrams which illustrate a method for adjusting a steering angle of an ultrasonic device based on a vector sum of a plurality of pieces of vector information relating to a region of interest of an object;
FIG. 7 is a flowchart which illustrates an ultrasonic image generating method according to an exemplary embodiment;
FIG. 8 is a flowchart which illustrates an ultrasonic image generating method according to another exemplary embodiment; and
FIG. 9 is a flowchart which illustrates an ultrasonic image generating method according to still another exemplary embodiment.

### DETAILED DESCRIPTION

Advantages and features of the present inventive concept, and implementation methods thereof will be clarified through the exemplary embodiments described below with reference to the accompanying drawings. The present inventive concept may, however, be embodied in different forms and should not be construed as limited to the exemplary embodiments set forth herein. Rather, these exemplary embodiments are provided for thoroughness and completeness. Further, the present disclosure is only defined by the scope of the claims. Like reference numerals refer to like elements throughout.

In the exemplary embodiments, the term "unit" refers to software and hardware components such as a field-programmable gate array (FPGA) and an application-specific integrated circuit (ASIC), and the "unit" performs operations. However, the "unit" is not limited to software or hardware. The "unit" may be configured to be included in an addressable storage medium, or may be configured to operate one or more processors. Therefore, for instance, the "unit" may include at least one of components such as software components, object-oriented software components, class components, and task components, and processes, functions, attributes, procedures, subroutines, program code segments, drivers, firmware, microcodes, circuits, data, database, data structures, tables, arrays, and variables. The components and functions provided in the "units" may be combined with each other to provide a smaller number of components and "units", or may be divided to provide additional components and "units".

Expressions such as "at least one of," when preceding a list of elements, modify the entire list of elements and do not modify the individual elements of the list.

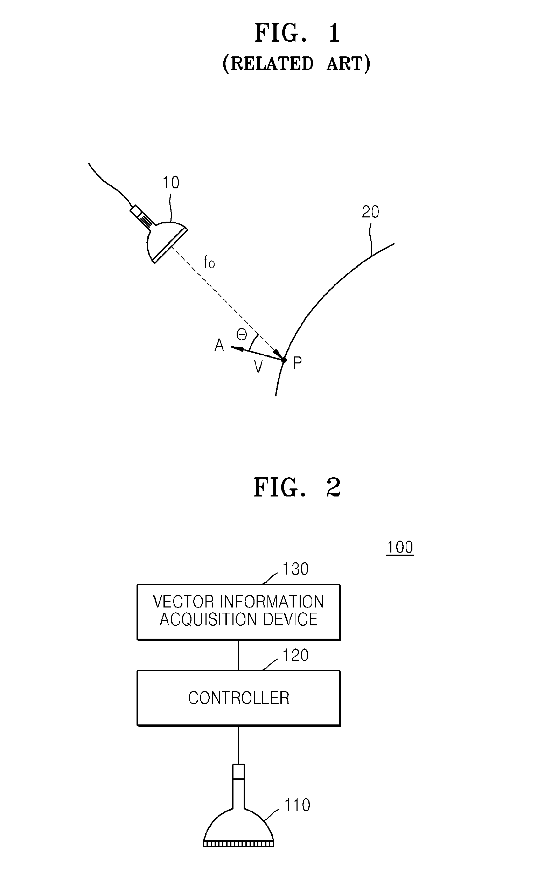

FIG. 1 is a diagram which illustrates a conventional method for obtaining vector information relating to an object 20.

In the present disclosure, "an object" may represent, for example, any one of a blood flow, an organ, or a specific portion in a body for which an ultrasonic image is to be obtained. In the present disclosure, "speed" is a scalar quantity and does not include a directional component.

A predetermined portion P of the object 20 moves at a speed of "v" in a direction "A." A probe 10 of an ultrasonic device transmits an ultrasonic signal having a frequency of f₀ to the predetermined portion P, and receives a response signal which is reflected from the predetermined portion P. Due to the Doppler effect, a frequency of the response signal varies based on the speed v of the predetermined portion P and an angle θ between a transmission direction of the ultrasonic signal and the movement direction of the predetermined portion P. In particular, a frequency difference between the ultrasonic signal transmitted to the predetermined portion P and the response signal is referred to as a Doppler frequency. A signal having the Doppler frequency is referred to as a Doppler signal. The speed of the predetermined portion P may be determined by using the Doppler frequency.

In the case where an angle between a movement direction of a particular portion and the ultrasonic signal transmitted to the object is 0°, a speed of each point may be accurately measured. However, it is difficult to accurately measure an actual speed of a portion that moves in other directions. In particular, in the case where the angle between a movement direction of a particular portion and the ultrasonic signal transmitted to the object is 90°, the Doppler frequency is 0 Hz, and thus the speed is measured to be zero.

Therefore, in order not to allow the angle between the ultrasonic signal transmitted to the object and the movement direction of the predetermined portion to be 90°, the movement direction of the predetermined portion is predicted so as to adjust a steering angle of the ultrasonic signal to be transmitted to the object.

According to the related art, in order to predict the movement direction of the object, a slope of a blood vessel is measured in order to predict a movement direction of blood flow. In general, a slope direction of the blood vessel corresponds to the direction of the blood flow. However, when a backward flow or a vortex flow occurs in the blood vessel, the direction of the blood flow does not correspond to the slope direction of the blood vessel. Therefore, it is difficult to accurately measure the speed of the blood flow by using the method of predicting the direction of the blood flow by measuring the slope of the blood vessel.

By using an ultrasonic device and an ultrasonic image generating method according to exemplary embodiments, the speed of the object may be accurately measured by automatically adjusting the steering angle of the ultrasonic signal based on vector information relating to the predetermined portion of the object.

FIG. 2 is a diagram which illustrates a configuration of an ultrasonic device 100 according to an exemplary embodiment.

Referring to FIG. 2, the ultrasonic device 100 according to an exemplary embodiment includes a probe 110, a controller 120, and a vector information acquisition device 130.

The probe 110 includes a plurality of elements, which plurality includes piezoelectric elements. The probe 110 transmits an ultrasonic signal to an object and receives a response signal which is reflected from the object. In particular, the probe 110 transmits the ultrasonic signal to a predetermined portion of the object and receives at least three first response signals which are respectively reflected from the predetermined portion of the object. Each of the at least three first response signals may have a respective Doppler frequency and a respective receiving angle.

The probe 110 may transmit the ultrasonic signal to the predetermined portion of the object multiple times and may receive the at least three first response signals which are reflected from the predetermined portion multiple times. For instance, when the three first response signals are respectively referred to as a 1-1 response signal, a 1-2 response signal, and a 1-3 response signal, the probe 110 receives, multiple times, each of the 1-1 response signal, the 1-2 response signal, and the 1-3 response signal. A plurality of 1-1 response signals, a plurality of 1-2 response signals, and a plurality of 1-3 response signals are respectively referred to as ensemble signals. In particular, the ensemble signals represent a plurality of response signals which are received at the same receiving angle. The use of ensemble signals improves the reliability of data obtained from the response signal by receiving the first response signal multiple times, thereby providing redundancy.

The controller 120 selects at least two first response signals from among the at least three first response signals received by the probe 110.

The controller 120 may select the at least two first response signals based on respective receiving angles of each of the received at least three first response signals. The receiving angle represents an angle between a direction of the first response signal and a line between the probe 110 and the predetermined portion of the object. In the case of selecting two first response signals from among the three first response signals, the controller 120 may select two first response signals, wherein an angle therebetween is greatest from among the angles formed by the three first response signals.

The controller 120 may select the at least two first response signals based on a respective power of each of the received at least three first response signals. The power represents the power of the corresponding Doppler signal. The controller 120 may select the at least two first response signals from among the three first response signals in descending order of the powers of the received at least three first response signals.

Because the power of a signal varies with the square of a frequency, it may be understood that the response signal having power of zero has been reflected at an angle of 90° with respect to the movement direction of the predetermined portion of the object. In particular, the first response signal which has a smallest power value is most perpendicular to the movement direction of the predetermined portion of the object, and the controller 120 may be configured to exclude the first response signal which is most perpendicular to the movement direction of the predetermined portion of the object. Such an exclusion may occur because a signal for which power is approximately equal to zero from among the at least three first response signals is blocked by a clutter filter (not illustrated) in the ultrasonic device 100, and thus meaningful information may not be obtainable from the signal.

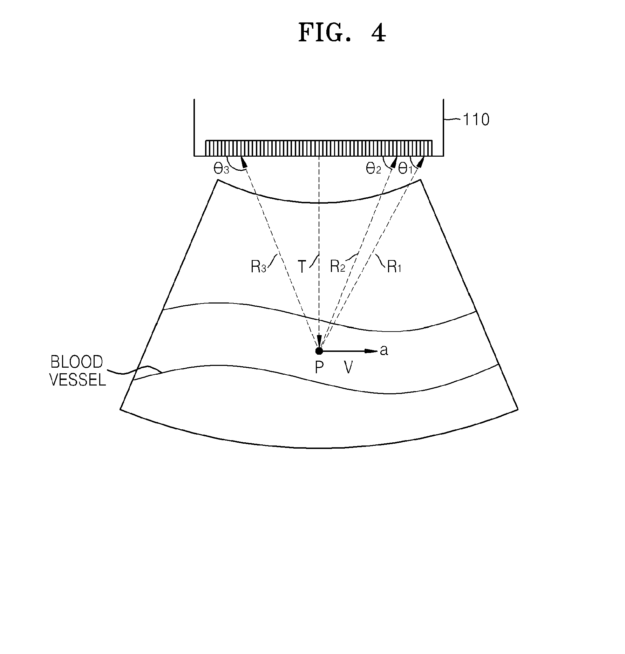

A method for selecting at least two first response signals from among the at least three first response signals is described below with reference to FIG. 4.

FIG. 4 is a diagram which illustrates a method for obtaining vector information relating to the predetermined portion of the object by using the ultrasonic device 100 according to an exemplary embodiment.

The probe 110 transmits an ultrasonic signal T to a predetermined portion P of a blood vessel. It is assumed that the predetermined portion P moves at a speed of "V" in a direction "a." The probe 110 receives three first response signals R₁, R₂, and R₃ which are reflected from the predetermined portion P. Receiving angles of each of the three first response signals R₁, R₂, and R₃ are respectively θ₁,θ₂, and θ₃. Because the predetermined portion P moves in the direction "a," a Doppler frequency of the first response signal R₁ has a smallest negative (-) value, a Doppler frequency of the first response signal R₂ has a negative (-) value which is greater than that of the first response signal R₁, and a Doppler frequency of the first response signal R₃ has a positive (+) value. Because the power of a signal varies with the square of a frequency, when an angle between the first response signal R₂ and the movement direction of the predetermined portion P is almost a right angle, the first response signal R₂ has the smallest power as compared with the power of response signal R₁ and the power of response signal R₃. The controller 120 may select the first response signals R₁ and R₃ from among the three first response signals R₁, R₂, and R₃, thereby excluding the first response signal R₂ which has the smallest power.

The controller 120 may select two first response signals, wherein an angle therebetween is greatest from among the angles formed by the three first response signals. In FIG. 4, the first response signals R₁ and R₃ are selected.

The vector information acquisition device 130 acquires vector information which indicates the speed and the movement direction of the predetermined portion P of the object based on the respective receiving angles and the respective Doppler frequencies of each of the at least two first response signals which are selected by the controller 120.

When the ensemble signals of the at least two response signals are acquired, the vector information acquisition device 130 may acquire the respective Doppler frequencies of each of the selected at least two response signals by using a respective autocorrelation of the ensemble signals, and may measure the speed and the movement direction of the predetermined portion P of the object by using the acquired Doppler frequencies.

It shall be apparent to those skilled in the art how the probe 110 acquires the vector information which indicates the speed and the movement direction of the predetermined portion of the object by using the respective receiving angles and the respective Doppler frequencies of each of the two response signals. Therefore, a detailed description of this operation is omitted.

The ultrasonic device 100 according to an exemplary embodiment may acquire the vector information which relates to each of a plurality of predetermined portions of the object in order to represent the object by generating a color Doppler image.

However, a relatively large amount of computations may be required in order to acquire the vector information relating to the plurality of predetermined portions of the object and to represent the acquired vector information by generating a color Doppler image by using a vector Doppler technique.

Therefore, an ultrasonic device 100 according to another exemplary embodiment may be used to acquire the vector information relating to the predetermined portion of the object, and then may generate a typical color Doppler image or a Doppler spectrum image by applying the acquired vector information.

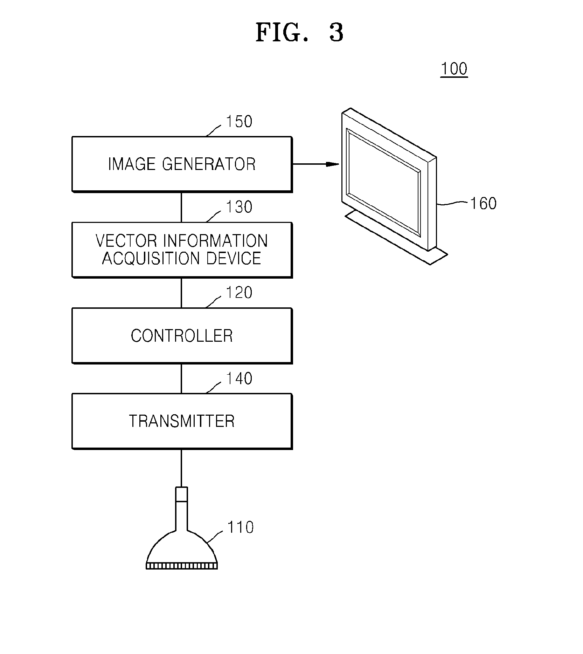

FIG. 3 is a diagram which illustrates a configuration of the ultrasonic device 100 according to another exemplary embodiment. Referring to FIG. 3, the ultrasonic device 100 according to another exemplary embodiment may include a probe 110, a controller 120, a vector information acquisition device 130, a transmitter 140, and an image generator 150, and/or a display 160. The probe 110, the controller 120, and the vector information acquisition device 130 have been described above in connection with the ultrasonic device 100 illustrated in FIG. 2. Therefore, detailed descriptions of these elements are omitted.

The transmitter 140 generates an ultrasonic signal based on a transmission control signal which is received from the controller 120. In particular, the transmitter 140 may analyze the transmission control signal which is received from the controller 120 in order to increase or decrease a beam width of the ultrasonic signal.

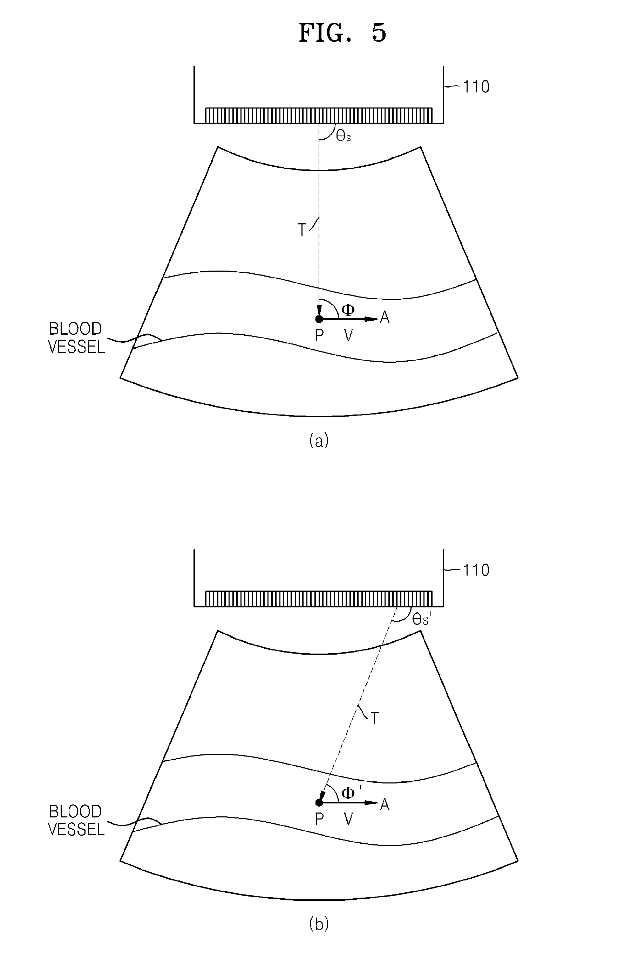

Further, the transmitter 140 adjusts, based on the vector information relating to the predetermined portion of the object which is acquired by the vector information acquisition device 130, a steering angle of the ultrasonic signal so that an angle between the ultrasonic signal transmitted to the object and the movement direction of the predetermined portion becomes a certain angle. The "steering angle" represents an angle between the ultrasonic signal transmitted to the object and the probe 110. The steering angle may be set so that the angle between the ultrasonic signal transmitted to the object and the movement direction of the predetermined portion becomes as close as possible to 0°. This will be described below in detail with reference to FIG. 5.

In FIG. 5, drawings (a) and (b) are diagrams which illustrate a method for adjusting the steering angle of the ultrasonic device 100 based on the vector information relating to the predetermined portion of the object.

The vector information relating to the predetermined portion of the object may be acquired by the ultrasonic device 100 illustrated in FIG. 2, or may be acquired by a typical ultrasonic device which acquires the vector information relating to the predetermined portion by using a response signal which is reflected from the predetermined portion of the object.

In FIG. 5, drawing (a) illustrates that the ultrasonic signal T is transmitted to the predetermined portion P in a blood vessel before adjusting the steering angle. The predetermined portion P moves at a speed of "v" in a direction "A." In drawing (a) of FIG. 5, the steering angle of the ultrasonic signal T is set to θₛ.

In FIG. 5, drawing (b) illustrates that, after changing the steering angle, the steering angle of the ultrasonic signal is adjusted to θₛ'.

Referring to drawings (a) and (b) of FIG. 5, an angle between the ultrasonic signal which is transmitted to the predetermined portion P and the movement direction of the predetermined portion P is changed from Φ to Φ'. In these drawings, Φ' is smaller than Φ. The speed of the predetermined portion P may be more accurately measured as the angle between the movement direction of the predetermined portion P and the ultrasonic signal becomes closer to 0°. Therefore, by using the ultrasonic signal which is transmitted at the steering angle Φ', the speed of the predetermined portion P may be more accurately measured.

The probe 110 retransmits the ultrasonic signal for generating at least one of a color Doppler image and a Doppler spectrum image to the object at the adjusted angle, and receives a second response signal which is reflected from the object.

The image generator 150 may generate at least one of a color Doppler image and a Doppler spectrum image of the object based on the second response signal which is received by the probe 110.

The display 160 displays, to an examiner, the at least one of the color Doppler image and the Doppler spectrum image which is generated by the image generator 150.

On a typical color Doppler image, a region of interest (ROI) is set on the object, and movement of the entire region is displayed. The ultrasonic device 100 according to an exemplary embodiment may acquire a movement direction with respect to the entire ROI set on the object in order to provide a more correct color Doppler image.

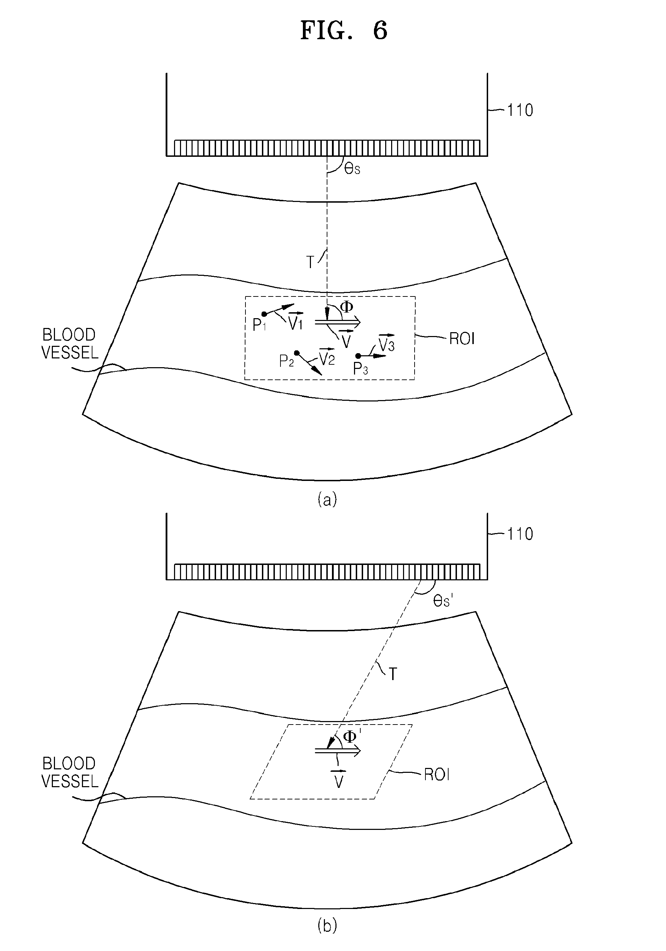

In particular, the probe 110 transmits the ultrasonic signal to each of a plurality of predetermined portions which are included in the ROI of the object, and receives at least three respective response signals from each of the predetermined portions. The controller 120 selects at least two response signals from among each respective grouping of the at least three received response signals, and the vector information acquisition device 130 acquires a plurality of pieces of vector information which respectively relate to each of the plurality of predetermined portions which are included in the ROI of the object. The vector information acquisition device 130 determines a vector sum of the plurality of pieces of vector information relating to the plurality of predetermined portions, and the transmitter 140 adjusts the steering angle of the ultrasonic signal so that an angle between the ultrasonic signal transmitted to the object and the movement direction of the vector sum becomes a certain angle. This will be described below in detail with reference to FIG. 6.

In FIG. 6, drawings (a) and (b) are diagrams which illustrate a method for adjusting the steering angle of the ultrasonic device 100 based on the vector sum which is determined in relation to the ROI of the object.

In FIG. 6, drawing (a) illustrates that the ultrasonic signal T is transmitted to the ROI in a blood vessel before changing the steering angle of the ultrasonic signal. The steering angle is set to θₛ. A first predetermined portion P₁ is associated with a vector V₁, a second predetermined portion P₂ is associated with a vector V₂, and a third predetermined portion P₃ is associated with a vector V₃. The vector sum of the vectors associated with the first predetermined portion P₁, the second predetermined portion P₂, and the third predetermined portion P₃ and which are acquired by the vector information acquisition determined 130 is computed and thus determined as a vector V.

In FIG. 6, drawing (b) illustrates that, after changing the steering angle, the steering angle of the ultrasonic signal is adjusted to θₛ', and accordingly, the ROI is changed.

Referring to drawings (a) and (b) of FIG. 6, an angle between the ultrasonic signal transmitted to the object and the movement direction of the vector sum is changed from Φ to Φ'. In these drawings, Φ' is smaller than Φ. The speed of the ROI of the object may be more accurately measured as the angle between the movement direction of the ROI of the object and the ultrasonic signal becomes closer to 0°. Therefore, by using the ultrasonic signal which is transmitted at the steering angle Φ', the speed of the ROI of the object may be more accurately measured.

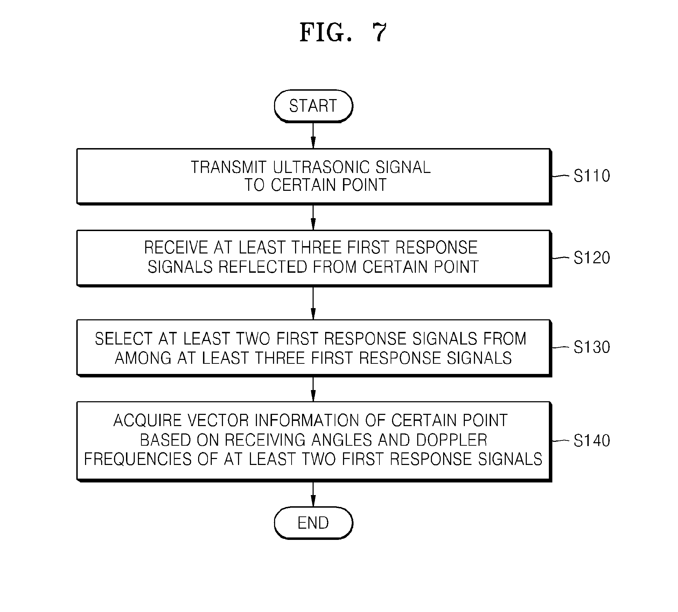

FIG. 7 is a flowchart which illustrates an ultrasonic image generating method according to an exemplary embodiment. Referring to FIG. 7, the ultrasonic image generating method according to an exemplary embodiment includes operations performed in a time series manner by using the ultrasonic device 100 illustrated in FIG. 2. Therefore, it may be understood that the above descriptions of the ultrasonic device 100 illustrated in FIG. 2 may be applied to the ultrasonic image generating method illustrated in FIG. 7 even though the descriptions are omitted below.

In operation S110, an ultrasonic device transmits an ultrasonic signal to a certain point or to a predetermined portion of an object.

In operation S120, the ultrasonic device receives at least three first response signals which are reflected from the certain point or to the predetermined portion of the object.

In operation S130, the ultrasonic device selects at least two first response signals from among the at least three received first response signals. The at least two first response signals may be selected based on at least one of a respective receiving angle and a respective power of each of the at least three received first response signals. In particular, the ultrasonic device may select the at least two first response signals from among the three first response signals in descending order of the respective powers of the at least three received first response signals.

In operation S140, the ultrasonic device may acquire vector information which indicates a speed and a movement direction of the certain point based on at least one of a respective receiving angle and a respective Doppler frequency of each of the selected at least two first response signals. In the case where ensemble signals of the selected at least two signals exist, the ultrasonic device may acquire the respective Doppler frequencies of each of the selected at least two first response signals by using a respective autocorrelation of the ensemble signals of each of the first response signals.

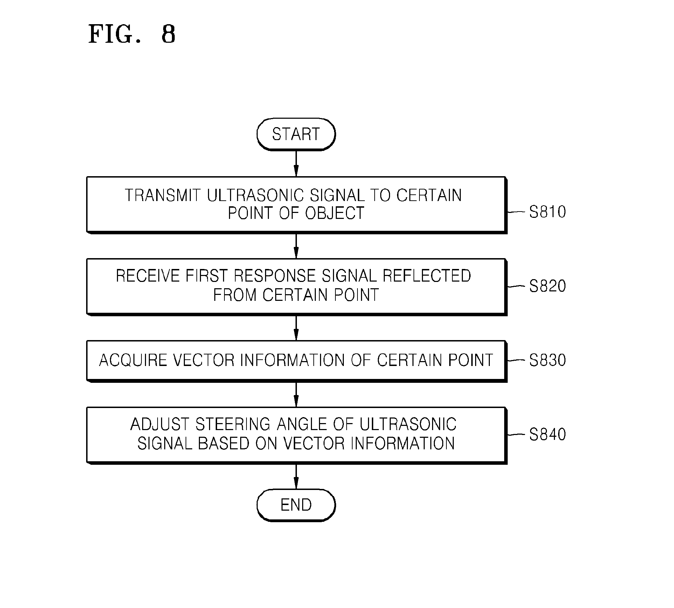

FIG. 8 is a flowchart which illustrates an ultrasonic image generating method according to another exemplary embodiment.

In operation S810, an ultrasonic device transmits an ultrasonic signal to a certain point or to a predetermined portion of an object.

In operation S820, the ultrasonic device receives a first response signal which is reflected from the certain point or from the predetermined portion of the object.

In operation S830, the ultrasonic device acquires vector information which indicates a speed and a movement direction of the certain point based on a receiving angle and a Doppler frequency of the received first response signal.

The vector information may be acquired by using the ultrasonic device 100 illustrated in FIG. 2, or may be acquired by using a typical ultrasonic device. In particular, the ultrasonic device may receive only two first response signals which are reflected from the certain point in order to acquire the vector information relating to the certain point by using the two received first response signals.

In operation S840, the ultrasonic device adjusts, based on the acquired vector information relating to the certain point, a steering angle of the ultrasonic signal so that an angle between the ultrasonic signal and a movement direction of the certain point becomes a certain angle.

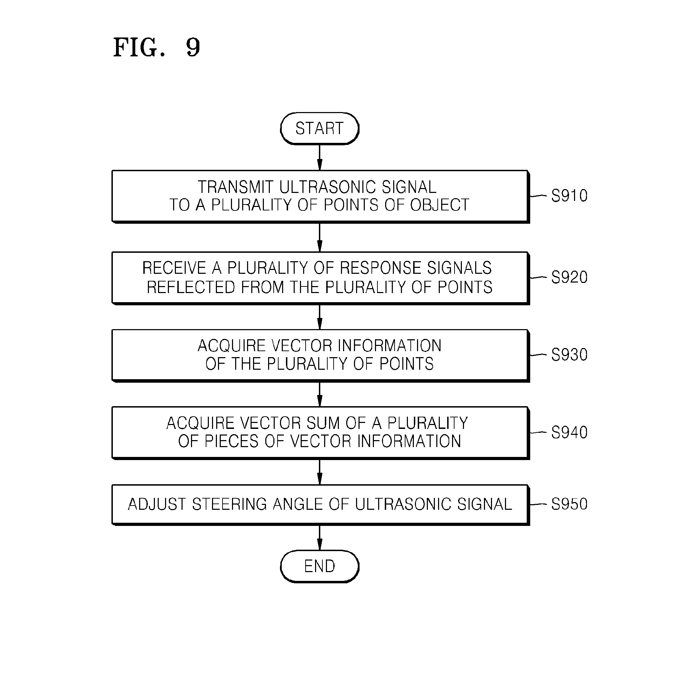

FIG. 9 is a flowchart which illustrates an ultrasonic image generating method according to another exemplary embodiment.

In operation S910, an ultrasonic device transmits an ultrasonic signal to each of a plurality of points or to each of a plurality of predetermined portions which are included in an ROI of an object.

In operation S920, the ultrasonic device receives a respective plurality of response signals which are respectively reflected from each of the plurality of points or from each of the plurality of predetermined portions which are included in the ROI of the object.

In operation S930, the ultrasonic device acquires a plurality of pieces of vector information which respectively indicate respective speeds and respective movement directions of each of the plurality of points based on respective receiving angles and respective Doppler frequencies of each of the plurality of received response signals.

The vector information may be acquired by using the ultrasonic device 100 illustrated in FIG. 2, or may be acquired by using a typical ultrasonic device. In particular, the ultrasonic device may receive only two first response signals which are respectively reflected from each of the plurality of points in order to acquire the vector information which respectively relates to each of the plurality of points by using the two first response signals.

In operation S940, the ultrasonic device determines a vector sum of the acquired plurality of pieces of vector information.

In operation S950, the ultrasonic device adjusts a steering angle of the ultrasonic signal based on the determined vector sum, so that an angle between the ultrasonic signal and a movement direction of the determined vector sum becomes a certain angle.

The above-described exemplary embodiments may be programmed to be executed by a computer, and may be implemented in a general digital computer which executes the program by using a transitory or a non-transitory computer-readable recording medium.

The computer-readable recording medium may include, for example, at least one of a magnetic storage medium (e.g., read-only memory (ROM), floppy disks, hard disks, and/or any other suitable type of magnetic storage medium), and an optical recording medium (e.g., compact disk - ROM (CD-ROMs) or digital versatile disks (DVDs)).

The ultrasonic device and the ultrasonic image generating method using vector Doppler, according to the exemplary embodiments described above, can be used to accurately measure vector information relating to an object.

Further, the ultrasonic device and the ultrasonic image generating method using vector Doppler, according to the exemplary embodiments described above, can be used to provide a reliable color Doppler image and/or a Doppler spectrum image to an examiner.

While the present inventive concept has been particularly shown and described with reference to exemplary embodiments thereof, it will be understood by those of ordinary skill in the art that various changes in form and details may be made therein without departing from the spirit and scope of the present disclosure as defined by the following claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method for generating an ultrasonic image, the method comprising:
transmitting an ultrasonic signal to a predetermined portion of an object (S110) and receiving at least three response signals which are respectively reflected from the predetermined portion (S120);
selecting at least two response signals from among the at least three received response signals (S130); and
acquiring vector information which indicates a speed and a movement direction of the predetermined portion based on a respective receiving angle and a respective Doppler frequency of each of the selected at least two response signals (S140).

2. The method of claim 1, wherein the selecting the at least two response signals comprises selecting the at least two response signals based on at least one of a respective receiving angle and a respective power of each of the at least three received response signals.

3. The method of claim 1 or claim 2, wherein the selecting the at least two response signals comprises selecting the at least two response signals from among the at least three response signals in a descending order of the powers of the at least three received response signals.

4. The method of any preceding claim, wherein the acquiring the vector information comprises acquiring the respective Doppler frequency of each of the selected at least two response signals by using a respective autocorrelation of ensemble signals for each of the selected at least two response signals.

5. The method of claim any preceding claim, further comprising:
adjusting, based on the acquired vector information, a steering angle of the ultrasonic signal.

6. The method of claim 5, further comprising:
transmitting the ultrasonic signal to the object at the adjusted steering angle and receiving a second response signal which is reflected from the object; and
generating at least one of a color Doppler image and a Doppler spectrum image of the object based on the received second response signal.

7. The method of claim 6, further comprising displaying the generated at least one of the color Doppler image and the Doppler spectrum image.

8. The method of claim 1,
wherein the transmitting of the ultrasonic signal comprises transmitting the ultrasonic signal to each of a plurality of predetermined portions which are included in a first region of interest of the object, and
wherein the acquiring of the vector information comprises acquiring a plurality of pieces of vector information which respectively indicate respective speeds and respective movement directions of each of the plurality of predetermined portions, and
the method further comprising:
determining a vector sum of the acquired plurality of pieces of vector information; and
adjusting a steering angle of the ultrasonic signal based on the determined vector sum.

9. The method of claim 8, further comprising determining a second region of interest of the object based on the adjusted steering angle.

10. An ultrasonic device (100) comprising:
a probe (110) which transmits an ultrasonic signal to a predetermined portion of an object and which receives at least three response signals which are respectively reflected from the predetermined portion;
a controller (120) which selects at least two response signals from among the at least three received response signals; and
a vector information acquisition device (130) which acquires vector information which indicates a speed and a movement direction of the predetermined portion based on a respective receiving angle and a respective Doppler frequency of each of the selected at least two response signals.

11. The ultrasonic device of claim 10,
wherein the controller adjusts, based on the acquired vector information, a steering angle of the ultrasonic signal.

12. The ultrasonic device of claim 11, wherein the probe transmits the ultrasonic signal to the object at the adjusted steering angle and receives a second response signal which is reflected from the object, and wherein the ultrasonic device further comprises an image generator which generates at least one of a color Doppler image and a Doppler spectrum image of the object by using the received second response signal.

13. The ultrasonic device of claim 10,
wherein the probe transmits the ultrasonic signal to each of a plurality of predetermined portions which are included in a first region of interest of the object and receives a respective plurality of response signals which are respectively reflected from each of the plurality of predetermined portions, and
wherein the vector information acquisition device acquires a plurality of pieces of vector information which respectively indicate respective speeds and respective movement directions of each of the plurality of predetermined portions based on respective receiving angles and respective Doppler frequencies of each of the plurality of received response signals, and determines a vector sum of the acquired plurality of pieces of vector information; and
wherein the controller adjusts a steering angle of the ultrasonic signal based on the determined vector sum.

14. The ultrasonic device of claim 13, wherein the controller determines a second region of interest of the object based on the adjusted steering angle.

15. A non-transitory computer-readable recording medium comprising a program which includes instructions for causing a computer to execute the ultrasonic image generating method of any one of claims 1 through 9.
